# EUROPEAN PATENT APPLICATION

(11) **EP 2 749 295 A1**
(43) Date of publication of application: **02.07.2014**
(21) Application number: 13199365.1
(22) Date of filing: 23.12.2013
(51) Int. Cl.: A61K 51/04, A61K 31/195, A61K 31/555, A61P 35/00

(54) **Radiopharmaceutical and pharmaceutical kit**

(30) Priority: 27.12.2012 JP 2012289455
(71) Applicant: NIHON MEDI-PHYSICS CO., LTD., Tokyo 136-0075 (JP); National Institute of Radiological Sciences, Chiba-shi, Chiba 263-8555 (JP); National Cancer Center, Tokyo 104-0045 (JP)
(72) Inventor: Yoshii, Yukie, Chiba-shi, Chiba 263-8555 (JP); Matsumoto, Hiroki, Sodegaura-shi Chiba 299-0266 (JP); Yoshimoto, Mitsuyoshi, Tokyo 104-0045 (JP)
(74) Representative: TBK

(57) **Abstract**

A radiopharmaceutical comprising a particular radioactive dithiosemicarbazone copper complex, wherein the radiopharmaceutical is used for combined administration with a chelating agent, and wherein the chelating agent comprises a multidentate ligand having a maximum dentate number of 2 or more to 4 or less.

## Description

This application is based on Japanese patent application No. 2012-289455, the content of which are incorporated hereinto by reference.

### BACKGROUND

### TECHNICAL FIELD

The present invention relates to a radiopharmaceutical and a pharmaceutical kit.

### RELATED ART

A radioactive dithiosemicarbazone copper complex is known as a diagnostic agent for hypoxic sites or mitochondrial dysfunction (for example, Japanese Patent Laid-Open No. H08-245425). Jason S. Lewis et al. (2001), Pros. Natl. Acad. Sci. vol. 98, 1206-1211 also discloses that a radioactive diacetyl-bis(N4-methylthiosemicarbazone) copper complex (hereinafter also referred to as "Cu-ATSM") is useful as a radiotherapeutic agent for tumor targeting hypoxic regions.

In recent years, it has also been revealed that ⁶⁴Cu-ATSM accumulates in CD133-positive cells (Yukie Yoshii et al. (2010), Nucl. Med. Biol. vol. 37, 395-404). Yukie Yoshii et al. (2011), Nucl. Med. Biol. vol. 38, 151-157 reports that it decreased the amount of CD133-positive cells in tumor to shrink the tumor. Thus, the radioactive Cu-ATSM is also expected to be useful as an agent for detecting cancer stem cells and as a preventive/therapeutic agent for cancer targeting cancer stem cells (Japanese Patent Laid-Open No. 2010-13380).

### SUMMARY

However, the administration of a radioactive dithiosemicarbazone copper complex to a body results in the accumulation of a large amount of radioactivity also in the normal liver; thus, the dose thereof has not been able to be sufficiently increased in view of avoiding exposure of the liver thereto. Thus, it has been difficult to obtain a clearer tumor image and to obtain a sufficient therapeutic effect against tumor, which has become one of problems for the practical use of the radioactive dithiosemicarbazone copper complex.

The present inventors have newly found that the combined administration of a radioactive dithiosemicarbazone copper complex and a particular chelating agent can promote the elimination of radioactivity from the liver.

Specifically, the present invention provides a radiopharmaceutical comprising a radioactive dithiosemicarbazone copper complex represented by general formula (1) below, wherein the radiopharmaceutical is used for combined administration with a chelating agent, and wherein the chelating agent comprises a multidentate ligand having a maximum dentate number of 2 or more to 4 or less. 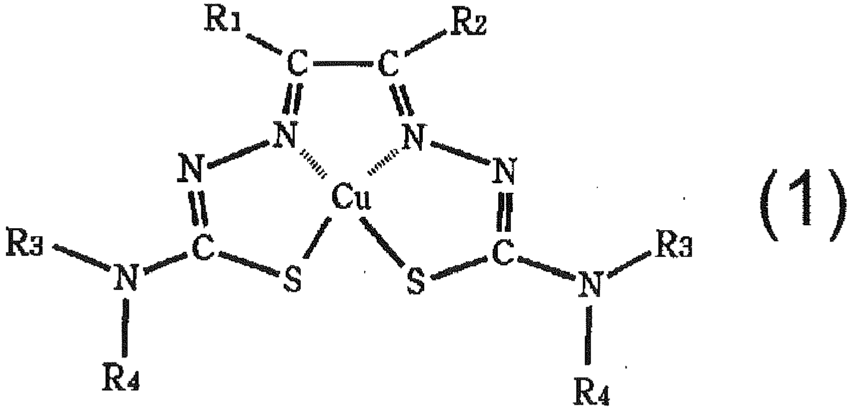 wherein R₁, R₂, R₃, and R₄ each independently represent a hydrogen atom, an alkyl group, or an alkoxy group, and Cu represents a radioactive isotope of copper atom.

According to the present invention, the combined use of a radioactive dithiosemicarbazone copper complex and a particular chelating agent can promote the elimination of radioactivity from the liver and thus can reduce exposure of the liver thereto upon administration of the radioactive dithiosemicarbazone copper complex.

### BRIEF DESCRIPTION OF THE DRAWINGS

The above and other objects, advantages and features of the present invention will be more apparent from the following description of certain preferred embodiments taken in conjunction with the accompanying drawing.

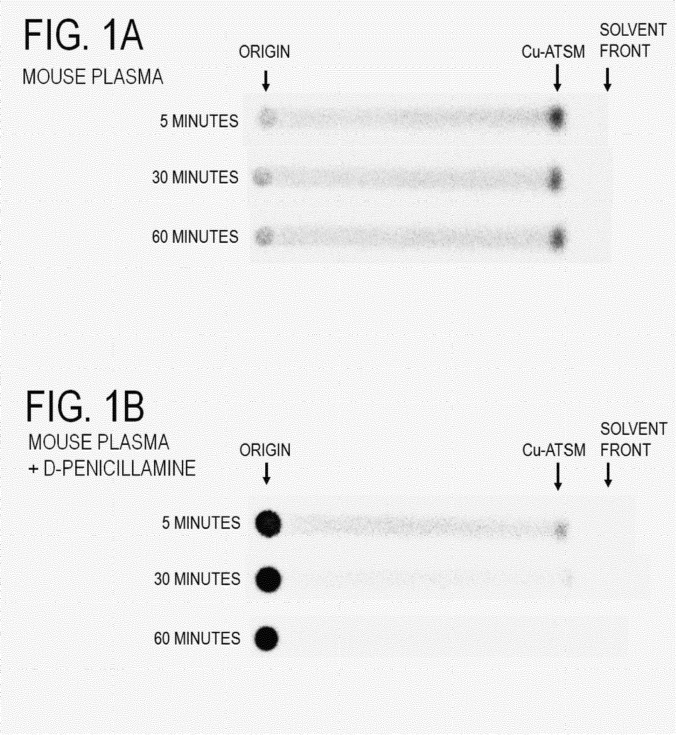

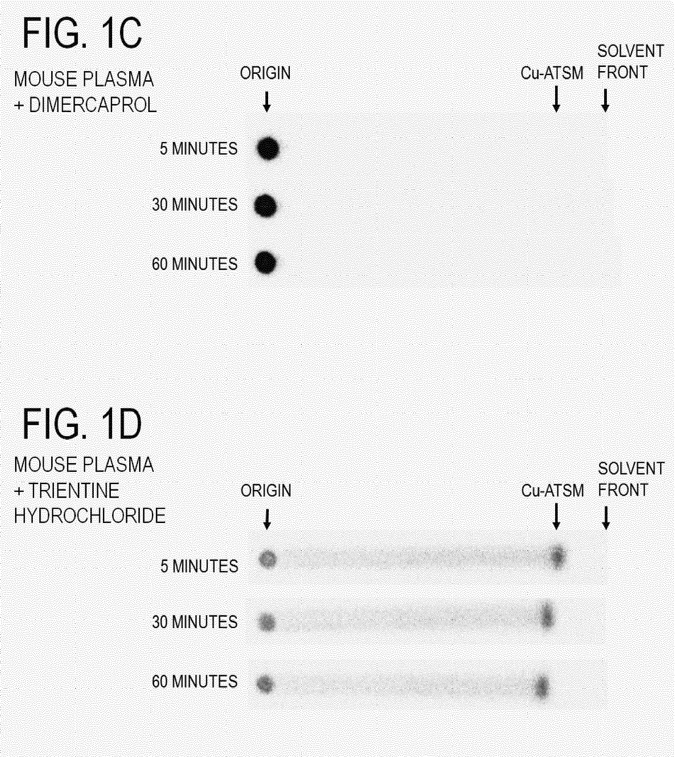

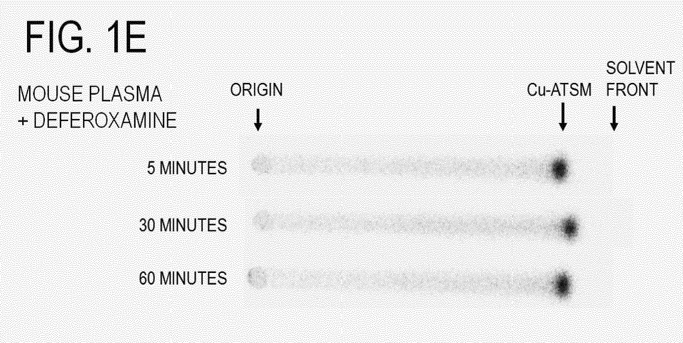

FIGs. 1A, 1B, 1C, 1D and 1E are a series of views showing the results of the ⁶⁴Cu complex exchange reaction between ⁶⁴Cu-ATSM and a chelating agent in mouse plasma confirmed by a thin-layer chromatography method. FIG. 1A is a view showing a result of reacting only mouse plasma as a control with ⁶⁴Cu-ATSM; FIG. 1B is a view showing a result of reacting ⁶⁴Cu-ATSM with D-penicillamine in mouse plasma; FIG. 1C is a view showing a result of reacting ⁶⁴Cu-ATSM with dimercaprol; FIG. 1D is a panel showing a result of reacting ⁶⁴Cu-ATSM with trientine hydrochloride; and FIG. 1E is a panel showing a result of reacting ⁶⁴Cu-ATSM with deferoxamine;

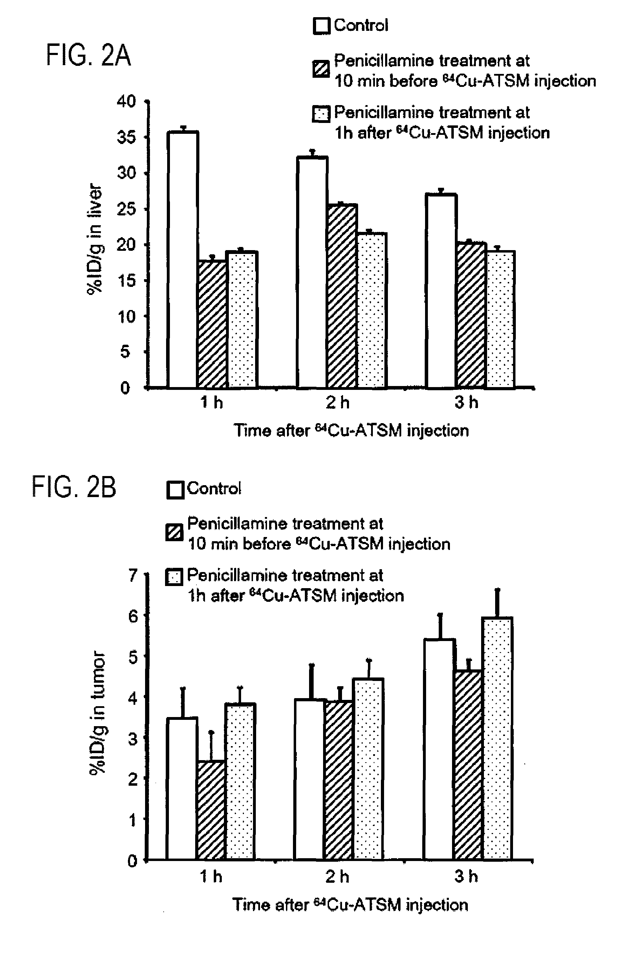

FIGs. 2A and 2B is a pair of graphs showing results of confirming the influence of D-penicillamine on the distribution of radioactivity upon administration of ⁶⁴Cu-ATSM in HT29-tumor bearing mice (500 mg/kg of D-penicillamine was administered 10 minutes before or 1 hour after the administration of ⁶⁴Cu-ATSM; a group receiving the administration of saline was included as a control; and FIG. 2A is a graph showing results in the liver and FIG. 2B is a graph showing results in the tumor);

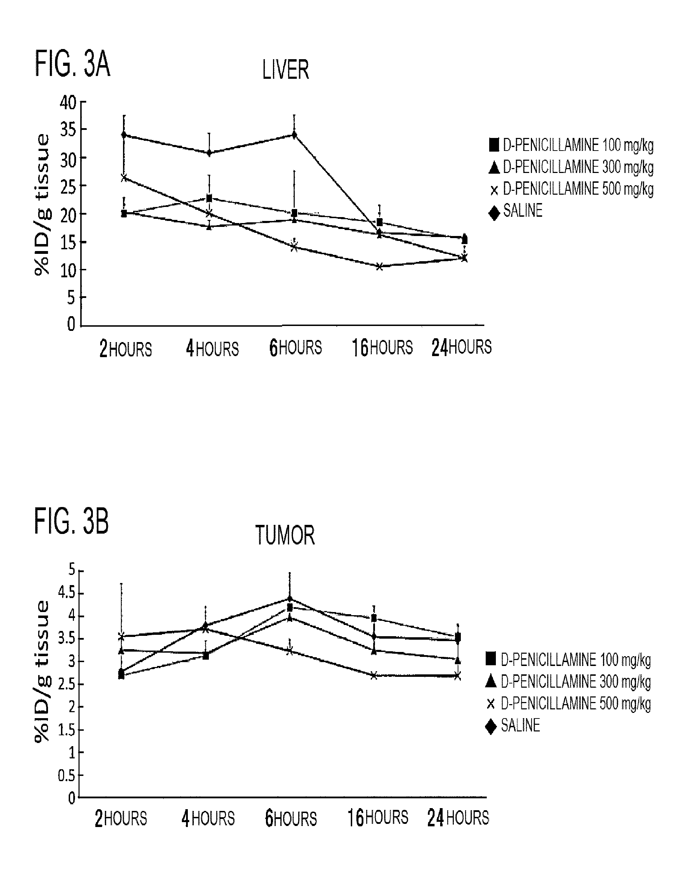

FIGs. 3A and 3B are a pair of graphs showing results of confirming the influence of 100 mg/kg, 300 mg/kg, and 500 mg/kg of D-penicillamine on the distribution of radioactivity upon administration of ⁶⁴Cu-ATSM in HT29-tumor bearing mice (a group receiving the administration of saline was included as a control; and FIG. 3A is a graph showing results in the liver and FIG. 3B is a graph showing results in the tumor);

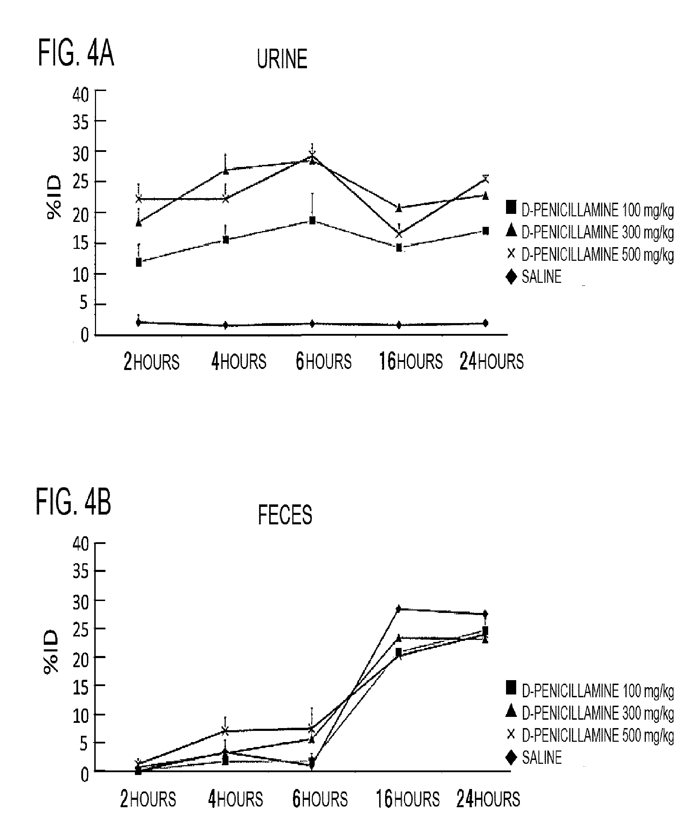

FIGs. 4A and 4B are a pair of graphs showing results of confirming the influence of 100 mg/kg, 300 mg/kg, and 500 mg/kg of D-penicillamine on the excretion of radioactivity upon administration of ⁶⁴Cu-ATSM in HT29-tumor bearing mice (a group receiving the administration of saline was included as a control; and FIG. 4A is a graph showing results of excretion into urine and FIG. 4B is a graph showing results of excretion into feces);

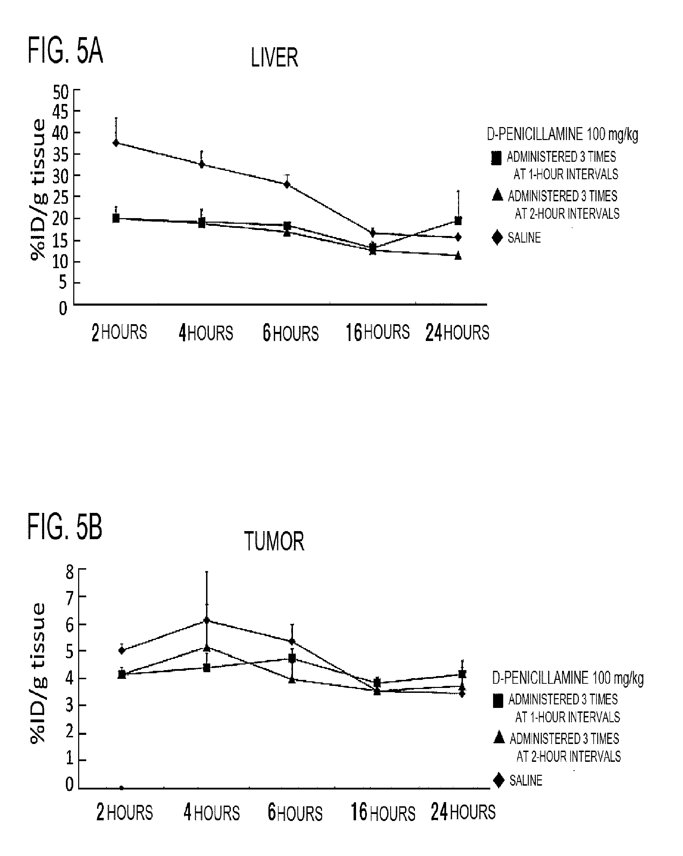

FIGs. 5A and 5B are a pair of graphs showing results of confirming the influence of the repeated administration of 100 mg/kg of D-penicillamine on the distribution of radioactivity upon administration of ⁶⁴Cu-ATSM in HT29-tumor bearing mice (each graph shows the comparison between 3 times at 1-hour intervals and 3 times at 2-hour intervals; a group receiving the administration of saline was included as a control; and FIG. 5A is a graph showing results in the liver and FIG. 5B is a graph showing results in the tumor);

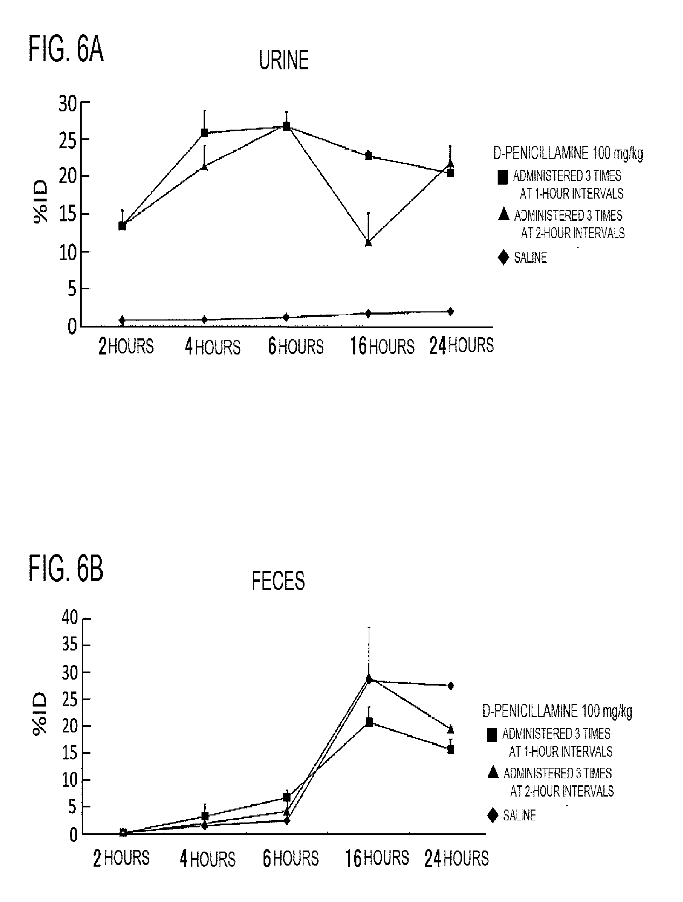

FIGs. 6A and 6B are a pair of graphs showing results of confirming the influence of the repeated administration of 100 mg/kg of D-penicillamine on the excretion of radioactivity upon administration of ⁶⁴Cu-ATSM in HT29-tumor bearing mice (each graph shows the comparison between 3 times at 1-hour intervals and 3 times at 2-hour intervals; a group receiving the administration of saline was included as a control; and FIG. 6A is a graph showing results of excretion into urine and FIG. 6B is a graph showing results of excretion into feces);

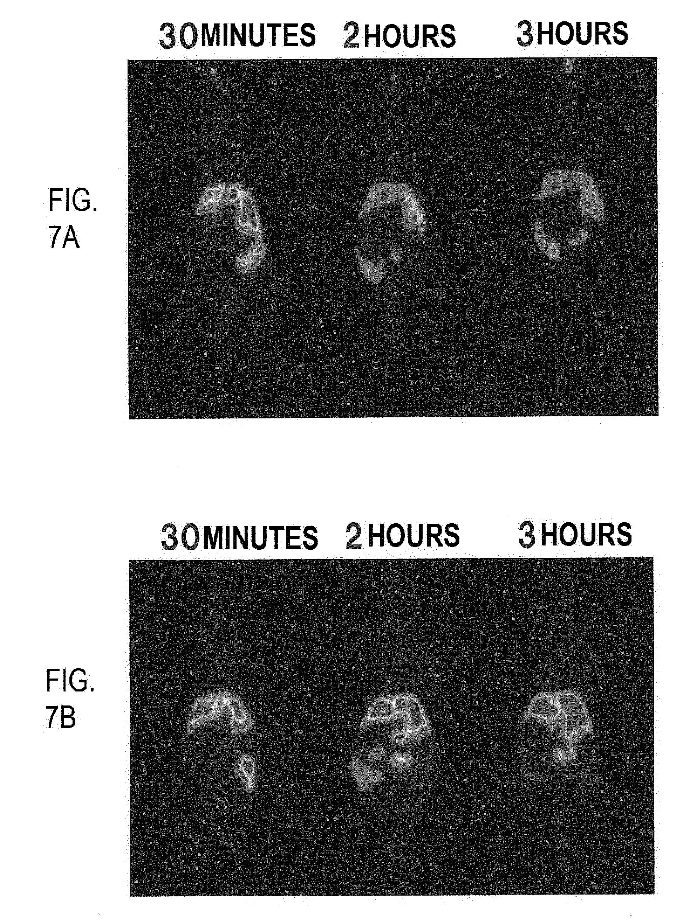

FIGs. 7A and 7B are a pair of series of photographs showing results of confirming the influence of 300 mg/kg of D-penicillamine on PET imaging upon administration of ⁶⁴Cu-ATSM using HT29-tumor bearing mice (FIG. 7A is a series of photographs showing results of whole-body PET imaging of a mouse receiving the administration of D-penicillamine, and FIG. 7B is a series photographs showing results of whole-body PET imaging of a mouse receiving the administration of saline as a control);

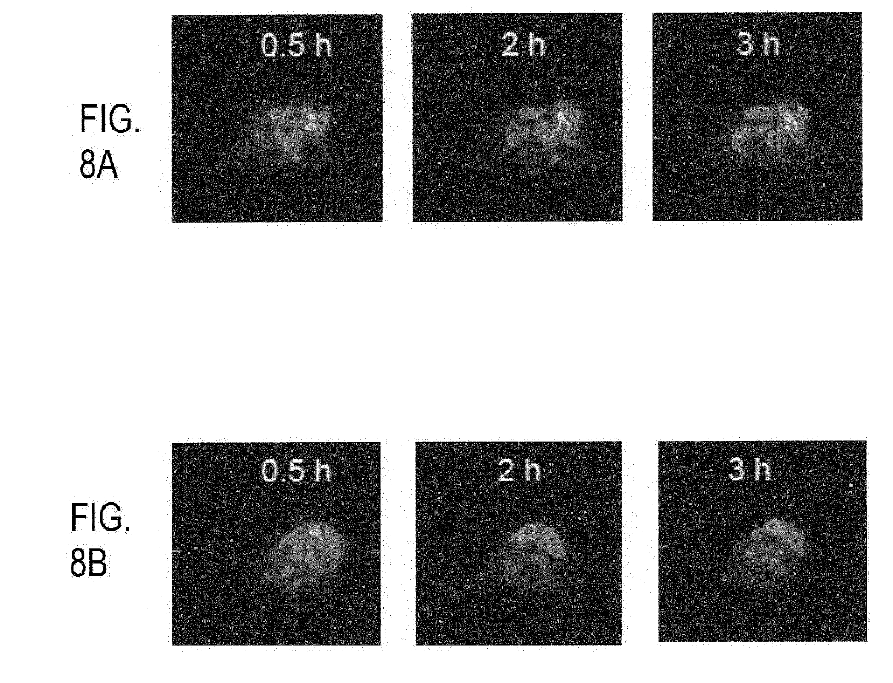

FIGs. 8A and 8B are a pair of series of photographs showing results of confirming the influence of 300 mg/kg of D-penicillamine on PET imaging upon administration of ⁶⁴Cu-ATSM using HT29-tumor bearing mice (FIG. 8A is a series of photographs showing results of PET imaging of a section containing tumor in a mouse receiving the administration of D-penicillamine, and FIG. 8B is a series of photographs showing results of PET imaging of a section containing tumor in a mouse receiving the administration of saline);

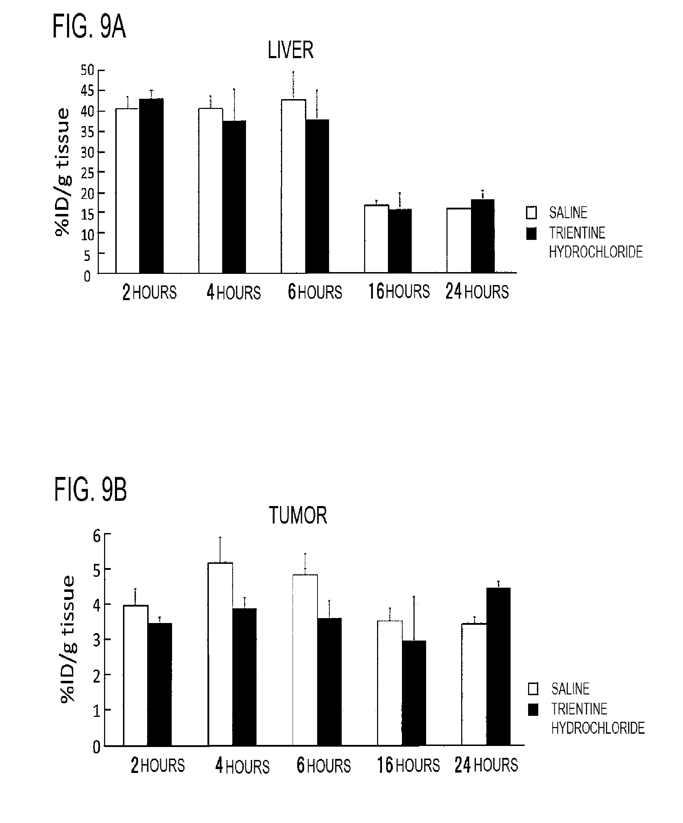

FIGs. 9A and 9B are a pair of graphs showing results of confirming the influence of 500 mg/kg of trientine hydrochloride on the distribution of radioactivity upon administration of ⁶⁴Cu-ATSM in HT29-tumor bearing mice (a group receiving the administration of saline was included as a control; and FIG. 9A is a graph showing results in the liver and FIG. 9B is a graph showing results in the tumor) ;

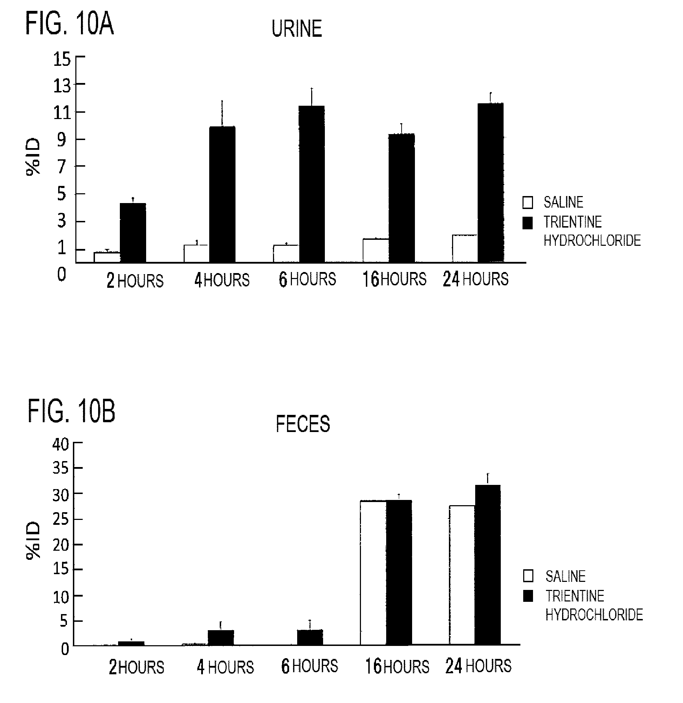

FIGs. 10A and 10B are a pair of graphs showing results of confirming the influence of 500 mg/kg of trientine hydrochloride on the excretion of radioactivity upon administration of ⁶⁴Cu-ATSM in HT29-tumor bearing mice (a group receiving the administration of saline was included as a control; and FIG. 10A is a graph showing results of excretion into urine and FIG. 10B is a graph showing results of excretion into feces);

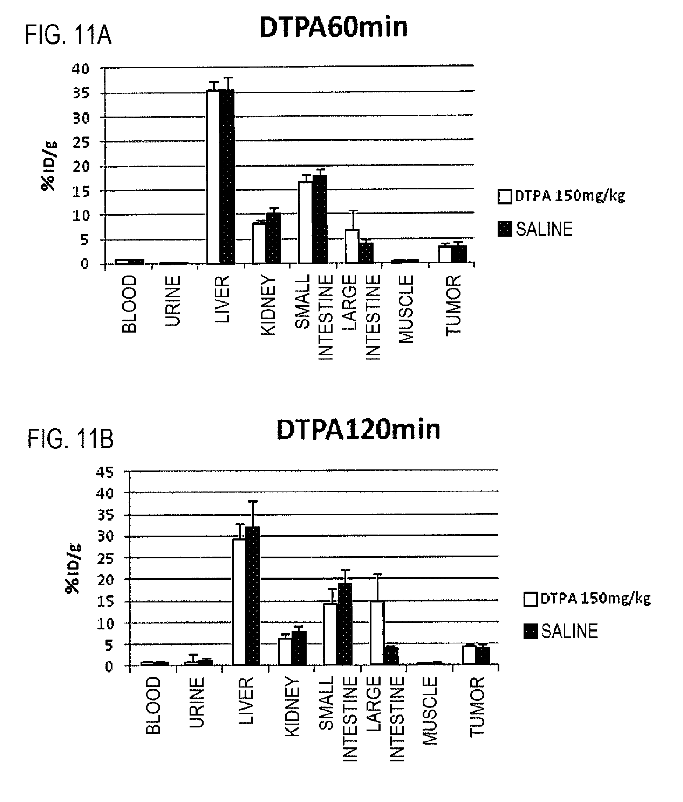

FIGs. 11A and 11B are a pair of graphs showing results of confirming the influence of 150 mg/kg of Ca-DTPA hydrochloride on the distribution and the excretion HT29-tumor bearing mice (a group receiving the administration of saline was included as a control; and FIG. 11A is a graph showing results at 1 hour after the administration of ⁶⁴Cu-ATSM and FIG. 11B is a graph showing results at 2 hours after the administration of ⁶⁴Cu-ATSM; and

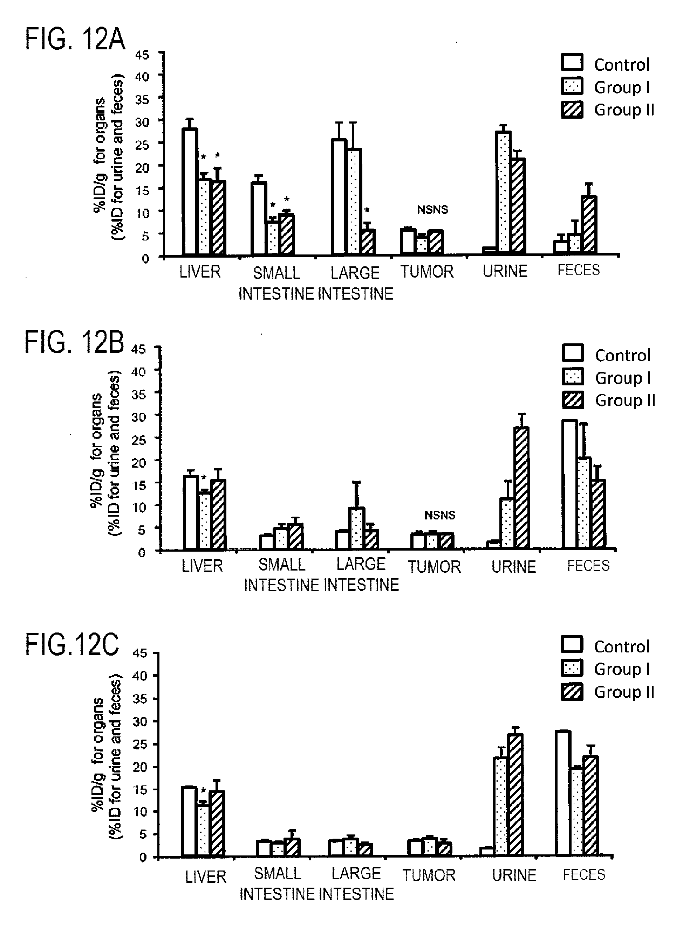

FIGs. 12A, 12B and 12C are a series of graphs showing results of confirming the influences of a group of 3 times repeated administration at 2-hour intervals of 100 mg/kg of D-penicillamine and a group further receiving glycerin enema on the distribution and the excretion of radioactivity upon administration of ⁶⁴Cu-ATSM in HT29-tumor bearing mice (a group receiving the administration of saline was included as a control; FIG. 12A is a graph showing results at 6 hours after the administration of ⁶⁴Cu-ATSM and FIG. 12B is a graph showing results at 16 hours after the administration of ⁶⁴Cu-ATSM; and FIG. 12C is a graph showing results at 24 hours after the administration of ⁶⁴Cu-ATSM.

### DETAILED DESCRIPTION

The invention will be now described herein with reference to illustrative embodiments. Those skilled in the art will recognize that many alternative embodiments can be accomplished using the teachings of the present invention and that the invention is not limited to the embodiments illustrated for explanatory purposed.

### [Radiopharmaceutical]

According to the present invention, the carbon atoms of the alkyl group and the alkoxy group for the substituents R₁, R₂, R₃, and R₄ in the general formula (1) are each preferably an integer of 1 to 5, more preferably an integer of 1 to 3. According to the present invention, it is preferable that the substituents R₁, R₂, R₃, and R₄ in the general formula (1) are identical or different and each is a hydrogen atom or an alkyl group having 1 to 3 carbon atoms; it is more preferable that R₁ and R₂ are identical or different and each is a hydrogen atom or an alkyl group having 1 to 3 carbon atoms, R₃ is a hydrogen atom, and R₄ is an alkyl group having 1 to 3 carbon atoms; and it is still more preferable that R₁ and R₂ are identical or different and each is a hydrogen atom or a methyl group, R₃ is a hydrogen atom, and R₄ is a methyl group.

Specific examples of a radioactive dithiosemicarbazone copper complex represented by the general formula (1) include
a radioactive glyoxal-bis(N4-methylthiosemicarbazone) copper complex,
a radioactive glyoxal-bis(N4-dimethylthiosemicarbazone) copper complex,
a radioactive ethylglyoxal-bis(N4-methylthiosemicarbazone) copper complex,
a radioactive ethylglyoxal-bis(N4-ethylthiosemicarbazone) copper complex,
a radioactive pyruvaldehyde-bis(N4-methylthiosemicarbazone) copper complex,
a radioactive pyruvaldehyde-bis(N4-dimethylthiosemicarbazone) copper complex,
a radioactive pyruvaldehyde-bis(N4-ethylthiosemicarbazone) copper complex,
a radioactive diacetyl-bis(N4-methylthiosemicarbazone) copper complex,
a radioactive diacetyl-bis(N4-dimethylthiosemicarbazone) copper complex, and
a radioactive diacetyl-bis(N4-ethylthiosemicarbazone) copper complex.
Among others, preferred is a radioactive diacetyl-bis(N4-methylthiosemicarbazone) copper complex (hereinafter also referred to as a radioactive Cu-ATSM) or a radioactive pyruvaldehyde-bis(N4-dimethylthiosemicarbazone) copper complex (hereinafter also referred to as a radioactive Cu-PTSM), and more preferred is a radioactive diacetyl-bis(N4-methylthiosemicarbazone) copper complex.

The radioactive isotope of copper atom in the general formula (1) is preferably ⁶¹Cu, ⁶²Cu, ⁶⁴Cu, or ⁶⁷Cu. These radioactive isotopes emit positrons. Radioactive dithiosemicarbazone copper complexes, preferably a radioactive Cu-ATSM accumulate in hypoxic regions. Also, the radioactive Cu-ATSM preferentially accumulates in cancer stem cell-rich regions including cancer stem cells themselves within tumors. Thus, a radiopharmaceutical containing ⁶¹Cu, ⁶²Cu, ⁶⁴Cu, or ⁶⁷Cu can be used as an agent for imaging tumor or ischemia, preferably tumor, using positron emission tomography (PET). ⁶⁴Cu and ⁶⁷Cu also emit β-rays having a short range, and has a therapeutic effect of destroying cells. Thus, a radiopharmaceutical containing ⁶⁴Cu or ⁶⁷Cu is more preferable as a therapeutic agent for tumor.

The radioactive dithiosemicarbazone copper complex can accumulate in various tumors. Examples of the tumors in which the radioactive dithiosemicarbazone copper complex accumulates include breast cancer, brain tumor, prostate cancer, pancreas cancer, stomach cancer, lung cancer, colon cancer, rectal cancer, large bowel cancer, small intestinal cancer, esophageal cancer, duodenal cancer, tongue cancer, pharyngeal cancer, salivary gland cancer, schwannoma, liver cancer, kidney cancer, bile duct cancer, endometrium cancer, uterocervical cancer, ovary cancer, bladder cancer, skin cancer, angioma, malignant lymphoma, malignant melanoma, thyroid cancer, parathyroid cancer, nasal cancer, paranasal cancer, bone tumor, angiofibroma, retinosarcoma, penis cancer, testis tumor, pediatric solid cancer, sarcoma, and leukemia. These tumors may be primary or metastatic.

The radiopharmaceutical of the present invention may be obtained by formulating the radioactive dithiosemicarbazone copper complex alone or together with a pharmacologically acceptable carrier, diluent, or excipient. Dosage form may be for oral administration or for parenteral administration; however, a dosage form for parenteral administration such as injection is preferable.

### [Method for Producing Radiopharmaceutical]

The radiopharmaceutical of the present invention can be produced, for example, by the following method.

First, a dithiosemicarbazone derivative is synthesized by a method as described in Petering et al. (Cancer Res., 24, 367-372, 1964). Specifically, an aqueous solution or 50% by volume ethanol solution of 1 mole of α-keto aldehyde is added dropwise to a 5% glacial acetic acid solution containing 2.2 moles of thiosemicarbazide, N4-methylthiosemicarbazide, N4-dimethylthiosemicarbazide, or the like at 50 to 60°C over a period of 30 to 40 minutes. During the dropwise addition, the reaction solution is stirred. After the end of dropwise addition, the resultant is allowed to stand at room temperature for several hours and then cooled to separate crystals. The crystals are dissolved in methanol and recrystallized for purification.

Subsequently, radioactive copper ions are produced. ⁶¹Cu ions can be obtained by forming ⁶¹Cu from the ⁵⁹Co (α, 2n) ⁶¹Cu reaction, ^{nat}Zn (p, x) ⁶⁷Cu reaction, ⁵⁸Ni (α, p) ⁶¹Cu reaction, or the like and then chemically separating it from the target using ion chromatography or the like. ⁶²Cu ions can also be obtained using a ⁶²Zn/⁶²Cu generator as described, for example, in WO2005/08416 or Journal of Nuclear Medicine, vol. 30, 1989, pp. 1838-1842. ⁶⁴Cu ions can be obtained, for example, by the method of McCarthy et al. (Nuclear Medicine and Biology, vol. 24(1), 1997, pp. 35-43) or the method of Obata et al. (Nuclear Medicine and Biology, vol. 30(5), 2003, pp. 535-539). ⁶⁷Cu ions can be obtained, for example, by forming ⁶⁷Cu from the ⁶⁸Zn (p, 2p) ⁶⁷Cu reaction and then chemically separating it from the target using ion chromatography or the like.

Thereafter, the dithiosemicarbazone derivative can be used in the form of a dimethyl sulfoxide (DMSO) solution and contacted with a solution containing the radioactive copper ions to provide a radioactive dithiosemicarbazone copper complex represented by the general formula (1). Methods for producing ⁶²Cu-dithiosemicarbazone copper complex include, for example, a method as described in Japanese Patent Laid-Open No. H08-245425. Methods for producing ⁶¹Cu-ATSM include, for example, the method of Jalilian et al. (Acta Pharmaceutica, 59(1), 2009, pp. 45-55). Methods for producing ⁶²Cu-ATSM include, for example, a method as described in "PET yo Houshaseiyakuzai no Seizo oyobi Hinshitsukanri - Gousei to Rinshoushiyou eno Tebiki (Production and Quality Control of Radiopharmaceuticals for PET - A Handbook for Synthesis and Clinical Use)" (edited by PET Kagaku Workshop (PET Chemistry Workshop)) Fourth Edition (Revised in 2011). Methods for producing ⁶⁴Cu-ATSM include, for example, the method of Tanaka et al. (Nuclear Medicine and Biology, vol. 33, 2006, pp. 743-50).

The radioactive dithiosemicarbazone copper complex thus produced can be formulated in the form of an injection by dissolving, suspending, or emulsifying it in an aqueous solvent or an oily solvent and, if necessary, adding additives such as a dispersant, preservative, isotonizing agent, solubilizing agent, suspending agent, buffer agent, stabilizer, soothing agent, and antiseptic agent.

The radiopharmaceutical of the present invention is used by administering it in combination with a chelating agent to be described later. For the purpose of the present invention, "combined administration" may be such administration that the radioactive dithiosemicarbazone copper complex results in the coexistence, in the body, with a multidentate ligand contained in the chelating agent. The radiopharmaceutical may be administered before the multidentate ligand is metabolized or excreted after the administration of the chelating agent, or the chelating agent may be administered before the radioactivity of the radioactive dithiosemicarbazone copper complex disappears after the administration of the radiopharmaceutical. The radiopharmaceutical and the chelating agent may also be simultaneously administered. In the case where the radiopharmaceutical of the present invention is used as an agent for imaging or therapeutic agent for tumor, the chelating agent is preferably administered after the administration of the radiopharmaceutical and is more preferably administered when the radioactivity distribution has reached equilibrium in the body. This can reduce the accumulation of radioactivity in the liver while maintaining the accumulation of radioactivity in tumor from the liver.

The radiopharmaceutical of the present invention may also be used in combination with an enema agent as well as the chelating agent, as will hereinafter be described. This can suppress the accumulation of radioactivity in the colon while promoting the elimination of radioactivity from the liver and promote the excretion of radioactivity through urine and feces.

### [Chelating Agent]

For the purpose of the present invention, the chelating agent is one comprising a multidentate ligand having a maximum dentate number of 2 or more to 4 or less; however, preferred is a chelating agent comprising a multidentate ligand having a maximum dentate number of 2 or 3. The maximum dentate number refers to the maximum number thereof capable of coordinating metal ions for each molecule.

The multidentate ligand contained in the chelating agent of the present invention preferably contains a nitrogen atom or a sulfur atom in the molecule, more preferably at least contains a sulfur atom, and still more preferably contains a nitrogen atom and a sulfur atom. The multidentate ligand may be an aromatic multidentate ligand or an aliphatic multidentate ligand; however, preferred is an aliphatic multidentate ligand. The multidentate ligand may also be a cyclic multidentate ligand or a linear multidentate ligand; however, preferred is a linear multidentate ligand.

The multidentate ligand of the present invention is preferably a linear aliphatic multidentate ligand containing a nitrogen atom or a sulfur atom in the molecule, more preferably a linear aliphatic multidentate ligand at least containing a sulfur atom in the molecule, and still more preferably a linear aliphatic multidentate ligand at least containing a sulfur atom and a nitrogen atom in the molecule.

The multidentate ligand contained in the chelating agent of the present invention is preferably one or more selected from ethylenediamine (maximum dentate number: 2), dimercaprol (maximum dentate number: 2), penicillamine (maximum dentate number: 3), trientine (maximum dentate number: 4), and salts thereof, more preferably one or more selected from penicillamine, dimercaprol, trientine, and salts thereof, more preferably one or more selected from penicillamine, dimercaprol, and salts thereof, and still more preferably penicillamine or a salt thereof. Penicillamine is preferably in D-form. When these multidentate ligands form salts, the salts may be any salts that are pharmaceutically acceptable.

The chelating agent in the present invention may be one approved and distributed as a pharmaceutical; examples thereof include Metalcaptase (R) (manufacture and distribution: Taisho Pharmaceutical Co., Ltd.), Bal (R) (manufacture and distribution: Daiichi Sankyo Co., Ltd.), and Metalite (R) (manufacture and distribution: Tsumura & Co.).

The chelating agent in the present invention may be obtained by formulating the multidentate ligand alone or together with a pharmacologically acceptable carrier, diluent, or excipient, and may have a dosage form suitable for oral administration or parenteral administration. Examples thereof include oral agents such as tablets, capsules, powders, granules, and syrups and parenteral agents such as injections, external preparations, suppositories, pellets, drops, and sustained release preparations. Preferred are oral agents or injections, more preferably oral agents. Two or more dosage forms may be combined; for example, an oral agent and an injection may be administered in combination.

The chelating agent may be administered singly or a plurality of times before or after the administration of the radiopharmaceutical. It may be administered both before and after the administration of the radiopharmaceutical. The chelating agent is preferably administered after the administration of the radiopharmaceutical, more preferably when the radioactivity distribution has reached equilibrium in vivo, and may also be administered a plurality of times at predetermined time intervals after the administration of the radiopharmaceutical. The chelating agent in the present invention may be used in combination with an enema, as will hereinafter be described.

### [Pharmaceutical Kit]

The pharmaceutical kit of the present invention has the radiopharmaceutical and the chelating agent; however, it preferably has the radiopharmaceutical comprising a radioactive Cu-ATSM and the chelating agent comprising a multidentate ligand having a maximum dentate number of 2 or more to 4 or less, more preferably has a combination of the radiopharmaceutical comprising a radioactive Cu-ATSM and the chelating agent comprising one or more multidentate ligands selected from of D-penicillamine, dimercaprol, and salts thereof, and still more preferably has a combination of the radiopharmaceutical comprising a radioactive Cu-ATSM and the chelating agent comprising D-penicillamine or a salt thereof.

The pharmaceutical kit of the present invention also preferably comprises a package insert informing that the chelating agent is administered after administering the radiopharmaceutical. The package insert more preferably discloses that the chelating agent is administered when the body distribution of the radiopharmaceutical has reached equilibrium after the administration of the radiopharmaceutical.

The pharmaceutical kit of the present invention may further comprise an enema agent. The combined use of the enema in addition to the chelating agent can reduce the accumulation of radioactivity in the colon while promoting the elimination of radioactivity from the liver and promote the excretion of radioactivity through urine and feces. The enema agent may be one comprising one or more selected from polyhydric alcohols such as glycerin and sorbitol, sodium salts such as sodium citrate and sodium hydrogen carbonate, and bisacodyl; however, preferred is one at least comprising glycerin.

### [Method for Using Pharmaceutical Kit]

A subject to which the radiopharmaceutical and chelating agent of the present invention are to be administered is, for example, a mammal, preferably a human. The doses of the radiopharmaceutical and chelating agent of the present invention vary depending on the type, age, sex, body weight, and symptoms of a subject or a patient to which they are to be administered, the method for administration, and the like, and are not particularly limited; however, as the dose of the radiopharmaceutical, the range thereof may be adopted which is generally adopted for typical radiopharmaceuticals. As the dose of the chelating agent, the range thereof may be adopted which is generally adopted for typical metal excretion agents. When the enema agent is used, the range of doses may be adopted which is generally used for enema agents.

In the case where the pharmaceutical kit of the present invention is used for diagnostic imaging, it is preferred that the chelating agent is administered when the body distribution of radioactivity has reached equilibrium after the administration of the radiopharmaceutical, followed by noninvasively detecting radioactive rays by positron emission tomography (PET) to image a part or whole of the body. Because the radioactive dithiosemicarbazone copper complex accumulates in a hypoxic region, a site in which radioactive rays are highly detected can be detected to diagnose ischemia and tumor; especially, a radioactive Cu-ATSM is excellent for the detection of cancer stem cell-rich regions. According to the present invention, the administration of a particular chelating agent can promote the elimination of radioactivity from the liver; thus, the exposure of the liver thereto can be reduced while obtaining a clear image by increasing the dose of the radioactive dithiosemicarbazone copper complex. An image having a clearer contrast between normal tissue and lesions can be obtained in the liver and the periphery thereof, making lesion diagnosis in the liver and peripheral organs simpler. The chelating agent can be administered when the body distribution of radioactivity has reached equilibrium after the administration of the radiopharmaceutical, to promote the elimination of radioactivity from the liver while maintaining the accumulation thereof in tumor, which enables the control of the dose and the acquisition of a clearer tumor image.

When the pharmaceutical kit of the present invention is used for the treatment of tumor, it is preferred that the chelating agent is administered when the body distribution of radioactivity has reached equilibrium after the administration of the radiopharmaceutical. In view of reliably obtaining a therapeutic effect, the administration of the single radiopharmaceutical or the combined administration of the radiopharmaceutical and the chelating agent may be repeated a plurality of times. This can provide therapeutic effects such as the suppression of proliferation or metastasis of tumor and the prevention or suppression of recurrence of cancer while preventing the exposure of the liver thereto. Especially, because Cu-ATSM accumulates in cancer stem cell-rich regions including cancer stem cells themselves within tumors, the administration of a radioactive Cu-ATSM can provide the effect of killing cancer stem cells.

In the case where the enema agent is used in combination with the chelating agent, the enema agent may be administered before the administration of the chelating agent or may be administered after the administration of the chelating agent; however, it is preferably administered after the administration of the chelating agent. The enema agent may also be administered before the administration of the radiopharmaceutical or may be administered after the administration of the radiopharmaceutical; however, it is more preferably administered after the administration of the radiopharmaceutical and still more preferably administered after the administration of the radiopharmaceutical and the chelating agent. The enema agent may be singly administered or may be administered a plurality of times at predetermined time intervals.

### EXAMPLES

The present invention will be described in further detail by describing Examples. However, the present invention is not intended to be limited to the contents thereof.

### (Example 1) Preparation of ⁶⁴Cu-ATSM and Various Chelating Agent Solution

### (Synthesis of ATSM)

The synthesis of diacetyl-bis(N4-methylthiosemicarbazone) (ATSM) was performed according to the method of Tanaka et al. (Nuclear Medicine and Biology, vol. 33, 2006, pp. 743-50).

### (Synthesis of ⁶⁴Cu-ATSM)

⁶⁴Cu was produced and purified according to the method of McCarthy et al. (Nuclear medicine and biology, vol. 24, 1997, pp. 35-43) and the method of Obata et al. (Nuclear medicine and biology, vol. 30, 2003, pp. 535-539). ⁶⁴Cu-ATSM was synthesized according to the method of Tanaka et al. (supra) by using ATSM and ⁶⁴Cu. The produced agent was tested using a thin layer chromatography method (TLC method), and one having a radiochemical purity of 95% or more was used for the following experiment. Analysis conditions of ⁶⁴Cu-ATSM using TLC are as follows.

TLC plate: silica gel plate (product name: Silica gel 60, from Merck Ltd. Japan)

Development phase: ethyl acetate

Detection: fluoroimage analyzer (Model: FLA-7000, from Fujifilm Corporation)

### (Preparation of Chelating Agent Solution)

D-penicillamine (from Tokyo Chemical Industry Co., Ltd.), dimercaprol (from Wako Pure Chemical Industries, Ltd.), trientine hydrochloride (from Tsumura & Co.), or deferoxamine mesylate (from Sigma-Aldrich Co. LLC) was properly dissolved in saline and used for the following experiment.

### (Example 2) Confirmation of ⁶⁴Cu Complex Exchange Reaction between ⁶⁴Cu-ATSM and Chelating Agent in Mouse Plasma

Blood collected from BALB/c nude mice (male, 6-week old, about 25 g in body weight, obtained from Japan SLC, Inc.) under diethyl ether anesthesia was centrifuged (high-speed cooling centrifuge Model MX-105, from Tomy Co., Ltd., 3,000 rpm, 10 minutes) to provide plasma. In the mouse plasma maintained at 37°C was mixed each of D-penicillamine, dimercaprol, trientine hydrochloride, and deferoxamine mesylate to a final concentration of 10 mg/mL, to which ⁶⁴Cu-ATSM was then added to a final concentration of 6 µCi/mL. After maintaining each of the mixtures at 37°C for 5 minutes, 30 minutes, or 60 minutes, methanol cooled at 4°C in advance was added in an equal amount to the plasma thereto, which was thoroughly mixed and centrifuged (as above), followed by analyzing the supernatant by a TLC method.

The results are shown in FIGs. 1A, 1B, 1C, 1D and 1E. FIG. 1A is a view showing the result of reacting only mouse plasma as a control with ⁶⁴Cu-ATSM; FIG. 1B is a view showing the result of reacting ⁶⁴Cu-ATSM with D-penicillamine in mouse plasma; FIG. 1C is a view showing the result of reacting ⁶⁴Cu-ATSM with dimercaprol; FIG. 1D is a view showing the result of reacting ⁶⁴Cu-ATSM with trientine hydrochloride; and FIG. 1E is a view showing the result of reacting ⁶⁴Cu-ATSM with deferoxamine. In the plasma containing each of D-penicillamine and dimercaprol, ⁶⁴Cu-ATSM and unidentified metabolites or degradation products rapidly disappeared, and the origin component increased. From these results, the ⁶⁴Cu exchange reaction from ⁶⁴Cu-ATSM to a highly polar complex was thought to proceed rapidly. Similar results were also obtained with trientine hydrochloride; however, the effect thereof was relatively weak. No complex exchange reaction by deferoxamine mesylate could be confirmed.

### (Example 3) Observation of Effect of D-Penicillamine on in vivo Pharmacokinetics and Excretion of ⁶⁴Cu-ATSM in HT29 Tumor-Bearing Mouse (1)

Human large bowel cancer-derived HT29 cells were purchased from ATCC and proliferated for use. The HT29 tumor-bearing model was prepared by implanting 1 x 10⁷ HT29 cells subcutaneously in the femoral region of BALB/c nude mice (male, 6-week old, about 25 g in body weight, obtained from Japan SLC, Inc.), and used for the experiment 3 weeks after implantation. The tumor-bearing mice were fasted from 16 hours or more before the start of the experiment. 185 kBq (5 µCi) of ⁶⁴Cu-ATSM was administered through the tail vein of the HT29 tumor-bearing mice, and D-penicillamine was orally administered to 500 mg/kg 10 minutes therebefore or 1 hour thereafter. Saline was administered to a control group in place of D-penicillamine. They were sacrificed by blood removal from the heart under diethyl ether anesthesia 1, 2, and 3 hours after the administration of ⁶⁴Cu-ATSM; each tissue was removed and weighed; and radioactivity was further measured using an automatic gamma counter (Model: 1480 Wizard 3, from PerkinElmer Co., Ltd.). The radioactivity distributed in each organ was expressed as radioactivity per g of organ (% ID (Injected Dose)/g tissue) when the amount administered is set to 100%.

The results are shown in FIGs. 2A and 2B. FIG. 2A is a graph showing the results in the liver and FIG. 2B is a graph showing the results in the tumor. FIGs. 2A and 2B were expressed in average and standard deviation for 4 mice. As shown in FIG. 2A, the uptake of radioactivity into the liver was markedly decreased by the oral administration of D-penicillamine. This result was not different between the oral administration of D-penicillamine before and after the administration of ⁶⁴Cu-ATSM. As shown in FIG. 2B, it was confirmed that while the oral administration of D-penicillamine before the administration of ⁶⁴Cu-ATSM decreased the uptake of radioactivity into the tumor, the oral administration of D-penicillamine 1 hour after the administration of ⁶⁴Cu-ATSM did not affect the uptake of ⁶⁴Cu-ATSM into the tumor. From these results, to reduce the accumulation thereof in the liver without affecting the uptake of ⁶⁴Cu-ATSM into the tumor, it was probably recommended that D-penicillamine be orally administered after the administration of ⁶⁴Cu-ATSM.

### (Example 4) Observation of Effect of D-Penicillamine on in vivo Pharmacokinetics and Excretion of ⁶⁴Cu-ATSM in HT29 Tumor-Bearing Mouse (2)

185 kBq (5 µCi) of ⁶⁴Cu-ATSM was administered through the tail vein of the HT29 tumor-bearing mice prepared by the same method as Example 3, and D-penicillamine was orally administered to 100, 300, or 500 mg/kg 1 hour thereafter. Saline was administered to a control group in place of D-penicillamine. They were sacrificed by blood removal from the heart under diethyl ether anesthesia 2, 4, 6, 16, and 24 hours after the administration of ⁶⁴Cu-ATSM; each tissue was removed and weighed; and radioactivity was further measured. The excreted urine and feces were recovered, and radioactivity was similarly measured. The radioactivity distributed in each organ was expressed as ID/g tissue. The excretion of radioactivity into each of urine and feces was expressed as radioactivity (% ID) when the amount administered was set to 100%.

The results are shown in FIGs. 3A, 3B, 4A and 4B. FIG. 3A is a graph showing the results in the liver and FIG. 3B is a graph showing the results in the tumor. FIG. 4A is a graph showing the results of excretion into urine and FIG. 4B is a graph showing the results of excretion into feces. FIGs. 3A, 3B, 4A and 4B were each expressed in average and standard deviation for 4 mice until 6 hours after administration and for 3 mice at 16 hours after administration and later. As shown in FIG. 3A, the uptake of radioactivity into the liver was dose-dependently decreased by the oral administration of D-penicillamine, and the decrease was statistically significant. As shown in FIG. 3B, the uptake of radioactivity into the tumor was confirmed to be not statistically significantly affected by the oral administration of D-penicillamine. As shown in FIG. 4A, the excretion of radioactivity into urine after administering ⁶⁴Cu-ATSM was confirmed to be statistically significantly promoted by the oral administration of D-penicillamine. As shown in FIG. 4B, the excretion of radioactivity into feces was less affected by D-penicillamine. As shown from these results, the oral administration of D-penicillamine decreased the uptake of ⁶⁴Cu-ATSM into the liver and resulted in the rapid excretion thereof mainly into urine while not affecting the uptake of ⁶⁴Cu-ATSM into tumor.

### (Example 5) Observation of Effect of D-Penicillamine on in vivo Pharmacokinetics and Excretion of ⁶⁴Cu-ATSM in HT29 Tumor-Bearing Mouse (3)

185 kBq (5 µCi) of ⁶⁴Cu-ATSM was administered through the tail vein of the tumor-bearing mice prepared by the same method as Example 3; D-penicillamine was orally administered to 100 mg/kg 1 hour thereafter; and D-penicillamine was then orally administered to 100 mg/kg 2 times at 1-hour or 2-hour intervals. Saline was administered to a control group in place of D-penicillamine. Thereafter, radioactivity in each organ, urine, and feces was measured as Example 4; the radioactivity distributed in each organ was expressed as % ID/g tissue, and the excretion of radioactivity into urine and feces was expressed as % ID.

The results are shown in FIGs. 5A, 5B, 6A and 6B. FIG. 5A is a graph showing the results in the liver and FIG. 5B is a graph showing the results in the tumor. FIG. 6A is a graph showing the results of excretion into urine and FIG. 6B is a graph showing the results of excretion into feces. FIGs. 5A, 5B, 6A and 6B were each expressed in average and standard deviation for 4 mice until 6 hours after administration and for 3 mice at 16 hours after administration and later. As shown in FIG. 5A, the uptake of radioactivity into the liver was decreased by the oral repeated administration of D-penicillamine at both 1-hour and 2-hour intervals. As shown in FIG. 5B, the uptake of radioactivity into the tumor was confirmed to be not statistically significantly affected by the oral repeated administration of D-penicillamine. As shown in FIG. 6A, the excretion of radioactivity into urine after administering ⁶⁴Cu-ATSM was confirmed to be statistically significantly promoted by the oral repeated administration of D-penicillamine. As shown in FIG. 6B, the excretion of radioactivity into feces was less affected by D-penicillamine. As shown from these results, like the single administration, the oral repeated administration of D-penicillamine decreased the uptake of ⁶⁴Cu-ATSM into the liver and resulted in the rapid excretion thereof mainly into urine while not affecting the uptake of ⁶⁴Cu-ATSM into tumor.

### (Example 6) Observation of Effect of D-Penicillamine on PET imaging of ⁶⁴Cu-ATSM in HT29 Tumor-Bearing Mouse

37 MBq (1 mCi) of ⁶⁴Cu-ATSM was administered through the tail vein of the tumor-bearing mice prepared by the same method as Example 3, and D-penicillamine was orally administered to 300 mg/kg 1 hour thereafter. The distribution of radioactivity was imaged using a PET device specific for small animals (Inveon, from Siemens Medical Systems, Inc.) 30 minutes and 2, 3, 4, 5, 6, 7, 8, and 24 hours after the administration of ⁶⁴Cu-ATSM. Image acquisition was performed for 5 minutes from each time point, and the image was reconstructed by a 3-dimensional maximum a posteriori probability method (3D-MAP method) using Inveon Acquisition Workplace Software (from Siemens Medical Systems, Inc.). As a control, PET imaging was also similarly performed in mice receiving the administration of saline in place of D-penicillamine.

The PET/CT images obtained are shown in FIGs. 7A, 7B, 8A and 8B. FIG. 7A shows the whole-body image of a mouse receiving the administration of D-penicillamine at 30 minutes, 2 hours and 3 hours respectively after the administration of ⁶⁴Cu-ATSM. FIG. 7B shows the whole-body image of a mouse receiving the administration of saline as a control at 30 minutes, 2 hours and 3 hours respectively after the administration of ⁶⁴Cu-ATSM. FIG. 8A shows the image of section containing tumor in a mouse receiving the administration of D-penicillamine at 30 minutes (0.5 h in FIG. 8A), 2 hours (2 h in FIG. 8A) and 3 hours (3 h in FIG. 8A) respectively after the administration of ⁶⁴Cu-ATSM. FIG. 8B shows the image of section containing tumor in a mouse receiving the administration of saline as a control at 30 minutes (0.5 h in FIG. 8B), 2 hours (2 h in FIG. 8B) and 3 hours (3 h in FIG. 8B) respectively after the administration of ⁶⁴Cu-ATSM. As shown, radioactivity accumulation in the liver was once observed in the mouse receiving the administration of ⁶⁴Cu-ATSM, but the administration of D-penicillamine was confirmed to decrease the accumulation of radioactivity in the liver and increase the accumulation of radioactivity in the bladder. The accumulation of radioactivity in the tumor was confirmed to be not affected by the administration of D-penicillamine.

### (Example 7) Observation of Effect of Trientine Hydrochloride on in vivo Pharmacokinetics and Excretion of ⁶⁴Cu-ATSM in HT29 Tumor-Bearing Mouse

185 kBq (5 µCi) of ⁶⁴Cu-ATSM was administered through the tail vein of the tumor-bearing mice prepared by the same method as Example 3, and trientine hydrochloride was orally administered to 500 mg/kg 1 hour thereafter. Saline was administered to a control group in place of trientine hydrochloride. Thereafter, radioactivity in each organ, urine, and feces was measured as Example 4; the radioactivity distributed in each organ was expressed as % ID/g tissue, and the excretion of radioactivity into urine and feces was expressed as % ID.

The results are shown in FIGs. 9A, 9B, 10A and 10B. FIG. 9A is a graph showing the results in the liver and FIG. 9B is a graph showing the results in the tumor. FIG. 10A is a graph showing the results of excretion into urine and FIG. 10B is a graph showing the results of excretion into feces. FIGs. 9A, 9B, 10A and 10B were each expressed in average and standard deviation for 4 mice until 6 hours after administration and for 3 mice at 16 hours after administration and later. As shown in FIG. 9A, the uptake of radioactivity into the liver tended to be decreased by the oral administration of trientine hydrochloride. As shown in FIG. 9B, the uptake of radioactivity into the tumor was confirmed to be not statistically significantly affected by the oral administration of trientine hydrochloride. As shown in FIG. 10A, the excretion of radioactivity into urine after administering ⁶⁴Cu-ATSM was confirmed to be statistically significantly promoted by the oral administration of trientine hydrochloride. As shown in FIG. 10B, the excretion of radioactivity into feces was less affected by trientine hydrochloride. As shown from these results, like D-penicillamine, trientine hydrochloride was confirmed to have the effect of promoting the excretion into urine; however, it was shown to weakly affect the liver compared to D-penicillamine.

### (Comparative Example 1) Observation of Effect of Ca-DTPA on in vivo Pharmacokinetics and Excretion of ⁶⁴Cu-ATSM in HT29 Tumor-Bearing Mouse

185 kBq (5 µCi) of ⁶⁴Cu-ATSM was administered through the tail vein of the tumor-bearing mice prepared by the same method as Example 3, and Ca-DTPA was administered to 150 mg/kg through the tail vein 10 minutes thereafter. Saline was administered to a control group in place of Ca-DTPA. Thereafter, radioactivity in each organ was measured as Example 3; the radioactivity distributed in each organ was expressed as % ID/g tissue.

The results are shown in FIGs. 11A and 11B. FIG. 11A is a graph showing the results at 1 hour after the administration of ⁶⁴Cu-ATSM and FIG. 11B is a graph showing the results at 2 hours after the administration of ⁶⁴Cu-ATSM. FIGs. 11A and 11B were expressed in average and standard deviation for 4 mice. As shown in FIGs. 11A and 11B, the uptake of radioactivity into each organ and tumor was confirmed to be statistically little affected by the intravenous administration of Ca-DTPA.

### (Example 8) Observation of Effect of D-Penicillamine on in vivo Pharmacokinetics and Excretion of ⁶⁴Cu-ATSM in HT29 Tumor-Bearing Mouse (4)

185 kBq (5 µCi) of ⁶⁴Cu-ATSM was administered through the tail vein of the tumor-bearing mice prepared by the same method as Example 3; D-penicillamine was orally administered to 100 mg/kg 1 hour thereafter; and D-penicillamine was then orally administered to 100 mg/kg 2 times at 2-hour intervals. In addition, a group was also provided in which 0.3 mL of glycerin enema solution (Glycerin Enema Solution 50% "Yoshida," Yoshida Pharmaceutical) was administered to the rectum 5.5 hours after the administration of ⁶⁴Cu-ATSM. Saline was administered to a control group in place of D-penicillamine. They were sacrificed by blood removal from the heart under diethyl ether anesthesia 6, 16, and 24 hours after the administration of ⁶⁴Cu-ATSM; each tissue was removed and weighed; and radioactivity was further measured. The excreted urine and feces were recovered, and radioactivity was similarly measured. The radioactivity distributed in each organ was expressed as ID/g tissue. The excretion of radioactivity into each of urine and feces was expressed as radioactivity (% ID) when the amount administered was set to 100%.

The results are shown in FIGs. 12A, 12B and 12C. Group I is a group in which D-penicillamine was orally administered to 100 mg/kg at each of 1, 3, and 5 hours after the administration of ⁶⁴Cu-ATSM, and Group II is a group in which D-penicillamine was orally administered to 100 mg/kg at each of 1, 3, and 5 hours after the administration of ⁶⁴Cu-ATSM, followed by performing glycerin enema after the administration of ⁶⁴Cu-ATSM. FIG. 12A shows radioactivity distribution at 6 hours after the administration of ⁶⁴Cu-ATSM; FIG. 12B shows radioactivity distribution at 16 hours after the administration of ⁶⁴Cu-ATSM; and FIG. 12C shows the distribution at 24 hours after the administration of ⁶⁴Cu-ATSM. FIGs. 12A, 12B and 12C were expressed in average and standard deviation for 4 mice. As shown in FIGs. 12A, 12B and 12C, it was confirmed that while the uptake of radioactivity into the liver was decreased by the oral repeated administration of D-penicillamine, the uptake of radioactivity into the tumor was not statistically significantly affected by the oral repeated administration of D-penicillamine. The excretion of radioactivity into urine after administering ⁶⁴Cu-ATSM was confirmed to be statistically significantly promoted by the oral repeated administration of D-penicillamine. Glycerin enema was performed after the oral repeated administration of D-penicillamine to reduce the accumulation of radioactivity in the colon to promote the excretion of radioactivity into urine and feces. These results showed that the combined use of the oral administration of D-penicillamine and glycerin enema can reduce the accumulation of radioactivity in the colon and promote the excretion of radioactivity through urine and feces while promoting the elimination of radioactivity from the liver.

The results of the above Examples showed that the combined administration of a radioactive dithiosemicarbazone copper complex such as a radioactive Cu-ATSM and a chelating agent comprising a multidentate ligand having a maximum dentate number of 2 or more to 4 or less (both inclusive) such as D-penicillamine, dimercaprol, or trientine hydrochloride can promote the elimination of radioactivity from the liver upon administration of the radioactive dithiosemicarbazone copper complex.

It is apparent that the present invention is not limited to the above embodiment, and may be modified and changed without departing from the scope and spirit of the invention.

A radiopharmaceutical comprising a particular radioactive dithiosemicarbazone copper complex, wherein the radiopharmaceutical is used for combined administration with a chelating agent, and wherein the chelating agent comprises a multidentate ligand having a maximum dentate number of 2 or more to 4 or less.

## Claims

1. A radiopharmaceutical comprising a radioactive dithiosemicarbazone copper complex represented by general formula (1) : 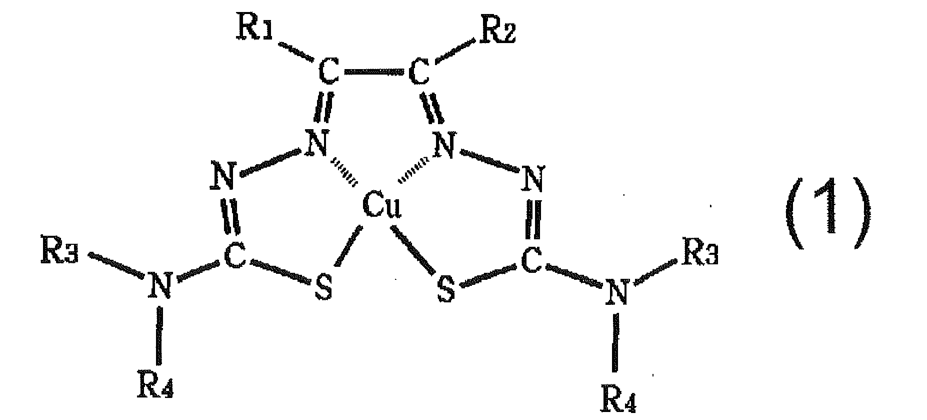 wherein R₁, R₂, R₃, and R₄ each independently represent a hydrogen atom, an alkyl group, or an alkoxy group, and Cu represents a radioactive isotope of copper atom,
wherein the radiopharmaceutical is used for combined administration with a chelating agent, and
wherein the chelating agent comprises a multidentate ligand having a maximum dentate number of 2 or more to 4 or less.

2. The radiopharmaceutical according to claim 1, wherein the chelating agent is to be administered after the administration of the radiopharmaceutical.

3. The radiopharmaceutical according to claim 1 or 2, wherein the radioactive isotope of copper atom is ⁶¹Cu, ⁶²Cu, ⁶⁴Cu, or ⁶⁷Cu.

4. The radiopharmaceutical according to any one of claims 1 to 3, wherein the radiopharmaceutical is a therapeutic agent for tumor.

5. The radiopharmaceutical according to any one of claims 1 to 3, wherein the radiopharmaceutical is an agent for imaging tumor.

6. The radiopharmaceutical according to any one of claims 1 to 5, wherein the radioactive dithiosemicarbazone copper complex is
a radioactive glyoxal-bis(N4-methylthiosemicarbazone) copper complex,
a radioactive glyoxal-bis(N4-dimethylthiosemicarbazone) copper complex,
a radioactive ethylglyoxal-bis(N4-methylthiosemicarbazone) copper complex,
a radioactive ethylglyoxal-bis(N4-ethylthiosemicarbazone) copper complex,
a radioactive pyruvaldehyde-bis(N4-methylthiosemicarbazone) copper complex,
a radioactive pyruvaldehyde-bis(N4-dimethylthiosemicarbazone) copper complex,
a radioactive pyruvaldehyde-bis(N4-ethylthiosemicarbazone) copper complex,
a radioactive diacetyl-bis(N4-methylthiosemicarbazone) copper complex,
a radioactive diacetyl-bis(N4-dimethylthiosemicarbazone) copper complex, or
a radioactive diacetyl-bis(N4-ethylthiosemicarbazone) copper complex.

7. The radiopharmaceutical according to any one of claims 1 to 6, wherein the multidentate ligand is one or more selected from D-penicillamine, dimercaprol, trientine, and salts thereof.

8. A pharmaceutical kit comprising:
a radiopharmaceutical comprising a radioactive dithiosemicarbazone copper complex represented by general formula (1) : 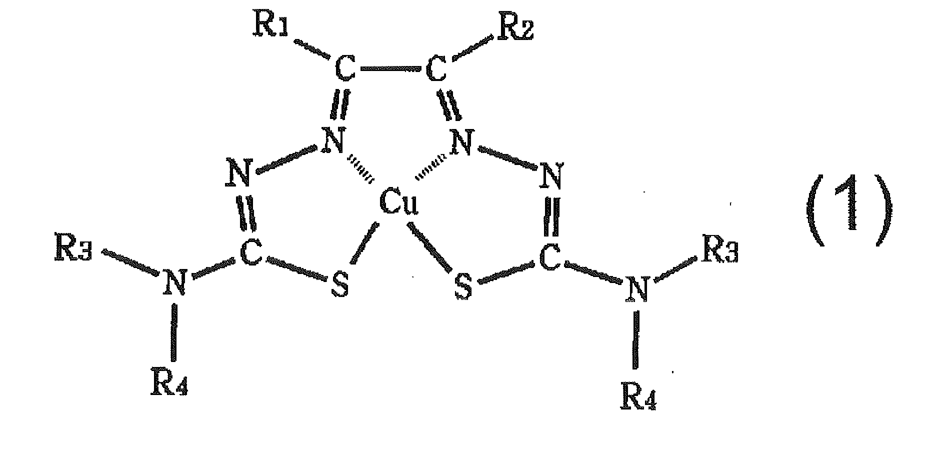 wherein R₁, R₂, R₃, and R₄ each independently represent a hydrogen atom, an alkyl group, or an alkoxy group, and Cu represents a radioactive isotope of copper atom; and
a chelating agent comprising a multidentate ligand having a maximum dentate number of 2 or more to 4 or less.

9. The pharmaceutical kit according to claim 8, further comprising an enema agent.

10. A chelating agent comprising a multidentate ligand having a maximum dentate number of 2 or more to 4 or less,
wherein the chelating agent is used for combined administration with a radiopharmaceutical, and
wherein the radiopharmaceutical comprises a radioactive dithiosemicarbazone copper complex represented by general formula (1): 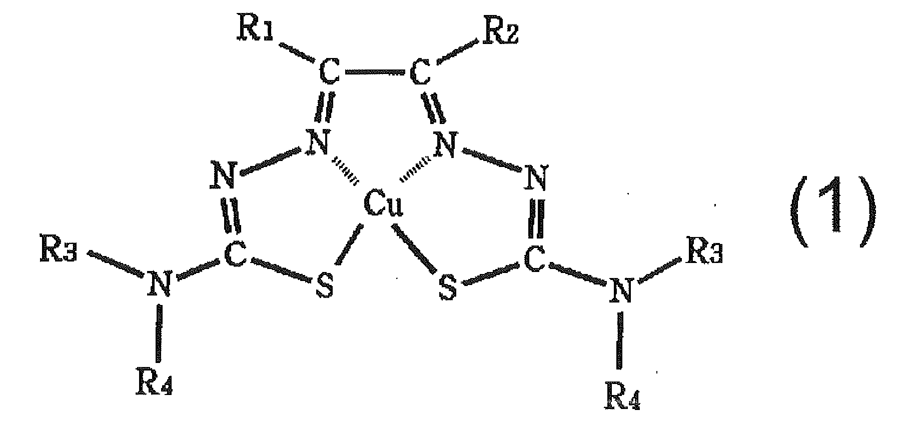 wherein R₁, R₂, R₃, and R₄ each independently represent a hydrogen atom, an alkyl group, or an alkoxy group, and Cu represents a radioactive isotope of copper atom.
